# EUROPÄISCHE PATENTSCHRIFT

(11) **EP 3 639 801 B1**
(45) Veröffentlichungstag und Bekanntmachung des Hinweises auf die Patenterteilung: **27.01.2021**
(21) Anmeldenummer: 19198392.3
(22) Anmeldetag: 19.09.2019
(51) Int. Cl.: A61F 13/49, B23K 26/384, B26F 1/31, B29D 7/01, C08J 5/18

(54) **ELASTISCHE FOLIE SOWIE VERFAHREN ZUR HERSTELLUNG EINES ELASTISCHEN SCHICHTMATERIALS**
ELASTIC FILM AND METHOD FOR PRODUCING ELASTIC COATING MATERIAL
FILM ÉLASTIQUE AINSI QUE PROCÉDÉ DE FABRICATION D'UN MATÉRIAU STRATIFIÉ ÉLASTIQUE

(30) Priorität: 17.10.2018 DE 102018125746
(43) Veröffentlichungstag der Anmeldung: 22.04.2020
(73) Patentinhaber: Mondi AG, 1030 Wien (AT)
(72) Erfinder: Brolund, Peer, 48599 Gronau (DE); Schwering, Ralf, 48565 Steinfurt (DE); Sollmann, Henner, 48599 Gronau (DE)
(74) Vertreter: Andrejewski - Honke Patent- und Rechtsanwälte Partnerschaft mbB

(56) Entgegenhaltungen:
- DE-A1-102012 203 755
- US-A- 5 336 554
- US-A1- 2001 008 676

## Beschreibung

Die Erfindung betrifft eine elastische Folie mit einer von Perforationslöchern gebildeten Perforation.

Der Gegenstand der vorliegenden Erfindung ist in den anhängenden Ansprüchen 1-15 definiert. Die elastische Folie ist insbesondere für die Herstellung von Einweg-Hygieneartikeln wie Babywindeln oder Inkontinenzartikeln für Erwachsene vorgesehen. Die elastische Folie wird dabei üblicherweise mit zumindest einer Deckschicht aus Nonwoven in geeigneter Weise kaschiert, um ein Laminat mit elastischen Rückstelleigenschaften zu bilden.

Babywindeln oder Inkontinenzartikel für Erwachsene weisen üblicherweise einen Grundkörper mit einer flüssigkeitsdichten Rückwand, einer flüssigkeitsdurchlässigen Innenwand und einer dazwischen angeordneten Saugeinlage auf. Um den Grundkörper im Hüftbereich des Benutzers halten zu können, sind dann im angelegten Zustand eine Rückseite sowie eine Vorderseite des Grundkörpers seitlich miteinander verbunden, wobei unterhalb dieser seitlichen Verbindung üblicherweise die Beinöffnungen für den Benutzer vorgesehen sind.

Das Laminat aus der elastischen Folie und zumindest einer Deckschicht aus Nonwoven kann insbesondere für die seitliche Verbindung eines solchen Hygieneartikels vorgesehen sein, wobei das Laminat für die Bildung einer Höschenwindel dauerhaft und unlösbar mit dem Grundkörper verbunden sein kann. Alternativ kann das Laminat auch in geeigneter Weise lediglich an der Vorderseite oder vorzugsweise der Rückseite des Grundkörpers befestigt sein und an seinem dann gegenüberliegenden freien Ende ein Verschlussmittel, beispielsweise ein Hakenmaterial aufzuweisen, um insbesondere zusammen mit einer entsprechenden Gegenfläche (landing zone) einen Klettverschluss zu bilden. Entsprechende Elemente mit einem elastische Rückstelleigenschaften aufweisenden Laminat werden in der Praxis auch als Windelohren bezeichnet.

Bei den beschriebenen Hygieneprodukten ergeben sich verschiedene spezifische Anforderungen. Da es sich um Wegwerfartikel handelt, sind möglichst geringe Gestehungskosten wünschenswert, sodass die einzelnen Komponenten mit möglichst wenig Materialeinsatz und auf möglichst einfache Weise gebildet sein sollen. Andererseits wird auch ein hoher Bedien- und Tragekomfort verlangt. Insbesondere müssen die beschriebenen Hygieneartikel an unterschiedliche Körperformen anpassbar sein und auch den Bewegungen eines Benutzers folgen. Dabei muss auch vermieden werden, dass die beschriebenen Hygieneartikel verrutschen oder auslaufen. Zu diesem Zweck werden üblicherweise elastische Folien mit einer großen Elastizität eingesetzt, sodass die gewünschten Haltekräfte erzeugt werden können, ohne dass es dabei zu unerwünschten Einschnürungen kommt.

Die elastische Folie kann dazu beispielsweise zumindest eine elastische Folienschicht aus Styrol-Block-Copolymer mit den üblichen Zusatzstoffen und Hilfsmitteln aufweisen.

Grundsätzlich kommen im Rahmen der Erfindung jedoch auch andere Materialien und Blends in Betracht, wobei der Begriff elastische Folie sich im Rahmen der Erfindung auf eine Folie bezieht, welche ohne ein Zerreißen ausgehend von einer Ausgangslänge in zumindest eine Richtung um zumindest 100 % dehnbar ist, wobei nach einem Wegfall der Zugkraft die verbleibende Verformung geringer als 25 % der Ausgangslänge ist. Übliche, auch im Rahmen der Erfindung geeignete, thermoplastische Elastomere weisen in der Praxis jedoch deutlich höhere Dehnbarkeiten und kleinere, bleibende Verformungen auf.

Die vorliegende Erfindung geht konkret von einer elastischen Folie mit einer von Perforationslöchern gebildeten Perforation aus, sodass durch die Perforation auch eine gute Atmungsaktivität sichergestellt werden kann. Gerade bei der zuvor exemplarisch beschriebenen Anwendung als Bestandteil eines Verschlussbandes von Hygieneartikeln ist eine solche Atmungsaktivität von besonderem Vorteil, um den Tragekomfort zu erhöhen und die Haut eines Benutzers vor Schweiß und Feuchtigkeit zu schützen, wodurch gerade bei einer kontinuierlichen Bewegung Hautreizungen oder wunde Stellen entstehen könnten.

Grundsätzlich kann eine solche Perforation auf unterschiedliche Weise gebildet werden. Eine gattungsgemäße elastische Folie ist aus der US 5,336,554 bekannt, wobei die Perforation mittels Laserstrahlung gebildet ist.

Auch die vorliegende Erfindung bezieht sich in besonderem Maße auf eine so gebildete Perforation, welche auch als Laserperforation bezeichnet werden kann. Eine Laserperforation zeichnet sich durch spezifische Merkmale aus und ist auch in der Praxis an dem gefertigten Produkt klar zu identifizieren.

Durch den Einsatz eines Lasers wird die elastische Folie aufgeschmolzen, wobei sich üblicherweise eine kreisrunde Öffnung ergibt, die von einer Wulst mit einer erhöhten Dicke umgeben ist. Diese Wulst bildet sich durch das von dem Laser aufgeschmolzene und danach wieder erstarrte Polymermaterial. Einerseits ist die Wulst aus zunächst aufgeschmolzenem und danach erstarrten Polymermaterial sehr gleichmäßig, sodass - anders als bei einer mechanisch erzeugten Perforation - keine bzw. weniger stark ausgeprägte lokale Schwachstellen vorliegen, welche ein Zerreißen begünstigen. Vielmehr ist es sogar so, dass durch die Materialanhäufung an der Wulst auf besonders vorteilhafte Weise eine Stabilisierung erfolgt. Die durch die Erzeugung der Perforation an sich eingebrachte Schwächung kann so gerade bei einer Laserperforation auf ein geringes Maß reduziert werden.

Gemäß der US 5,336,554 wird hervorgehoben, dass durch die beschriebene Perforation hinsichtlich eines Weiterreißens sogar eine Verbesserung gegenüber einer unperforierten Folie erreicht wird.

Eine gattungsgemäße elastische Folie ist des Weiteren auch aus der EP 1 598 172 A1 bekannt.

Auch wenn gerade bei einer Laserperforation vergleichsweise gute mechanische Eigenschaften erzielt werden können, führt die Perforation gerade im Hinblick auf die maximale Bruchdehnung zu einer Verschlechterung gegenüber einer unperforierten Folie.

Vor diesem Hintergrund liegt der vorliegenden Erfindung die Aufgabe zugrunde, die mechanischen Eigenschaften der elastischen Folie zu verbessern und ein größeres Anwendungsspektrum bzw. einen höheren Komfort für einen Benutzer zu ermöglichen.

Des Weiteren soll auch ein Verfahren zur Herstellung eines entsprechenden elastischen Schichtmaterials mit zumindest der elastischen Folie angegeben werden.

Gegenstand der Erfindung und Lösung der Aufgabe sind eine elastische Folie gemäß Patentanspruch 1 sowie ein Verfahren zur Herstellung eines elastischen Schichtmaterials gemäß Patentanspruch 11.

Ausgehend von einer elastischen Folie mit einer von Perforationslöchern gebildeten Perforation, insbesondere Laserperforation, ist erfindungsgemäß vorgesehen, dass die Perforationslöcher in einem ungedehnten Zustand entlang einer bevorzugten Dehnrichtung langgezogen sind, wobei das Verhältnis der entlang der bevorzugten Dehnrichtung bestimmten Länge zu einer senkrecht dazu bestimmten Breite der Perforationslöcher zumindest 3:2 beträgt und dass die Bruchdehnung entlang der bevorzugten Dehnrichtung zumindest doppelt so groß ist, wie die senkrecht dazu entlang der Breite der Perforationslöcher bestimmte Bruchdehnung.

Gemäß dem Stand der Technik werden mittels Laser im Wesentlichen runde Perforationslöcher erzeugt. Dies ist auch darauf zurückzuführen, dass bei dem Auftreffen des Laserstrahls das Polymermaterial der elastischen Folie schmilzt und sich ausgehend von dem Auftreffpunkt zurückzieht, wobei gerade die bei einer Laserperforation typische und im Hinblick auf die mechanischen Eigenschaften vorteilhafte Wulst gebildet wird.

Vor diesem Hintergrund ist jedoch gemäß der vorliegenden Erfindung eine langgezogene Form der Perforationslöcher mit einem Aspektverhältnis von zumindest 3:2 vorgesehen. Eine solche langgezogene Form kann dadurch erreicht werden, dass zur Erzeugung jedes Perforationsloches ein zugeordneter Laserstrahl in einer Relativbewegung über beispielsweise eine Länge von zumindest 200 µm über die elastische Folie bewegt wird.

Erfindungsgemäß sind die Perforationslöcher entlang der bevorzugten Dehnrichtung in der beschriebenen Weise langgezogen. Bei einer elastischen Folie ist gerade bei den eingangs beschriebenen Anwendungsfällen im Vorhinein bekannt, in welche Richtung nachfolgend eine Dehnung erfolgen soll. Erfindungsgemäß ist auch vorgesehen, dass die Bruchdehnung entlang der bevorzugten Dehnrichtung zumindest doppelt so groß ist, wie die senkrecht dazu entlang der Breite der Perforationslöcher bestimmte Bruchdehnung. Die Bruchdehnung (elongation at break) wird dabei ausgehend von einer Ausgangslänge in Prozent angegeben. Bei der Bruchdehnung (elongation at break) handelt es sich um einen üblichen, charakteristischen Parameter von Folien und insbesondere elastischen Folien. Die vorliegende Erfindung bezieht sich hinsichtlich der Bestimmung der Bruchdehnung auf die Norm ASTM 882-12. Dabei ist zu berücksichtigen, dass die Bruchdehnung sich gemäß der üblichen Nomenklatur auf die Gesamtlänge eines entsprechenden Folienmusters und nicht nur auf die Längenzunahme bei einer Dehnung bezieht.

Der vorliegenden Erfindung liegt in diesem Zusammenhang die Erkenntnis zugrunde, dass durch die entlang der bevorzugten Dehnungsrichtung langgezogenen Perforationslöcher die elastische Folie weniger stark geschwächt ist als senkrecht zu der bevorzugten Dehnrichtung. Bereits aufgrund geometrischer Betrachtungen wird deutlich, dass durch die langgezogene Form die elastische Folie in Bezug auf die bevorzugte Dehnrichtung weniger geschwächt ist. In Gedanken könnte beispielsweise ein breiter, in Dehnrichtung verlaufender Streifen der elastischen Folie durch eine Vielzahl schmalerer Streifen ersetzt werden, wobei dann mit einem ähnlichen elastischen Verhalten zu rechnen ist. Sofern solche Streifen dann senkrecht zu der bevorzugten Dehnrichtung durch lediglich schmale Stege untereinander verbunden sind, würde sich folglich auch senkrecht zu der bevorzugten Dehnrichtung nur eine geringe Festigkeit und somit auch nur eine geringe Bruchdehnung ergeben.

Das Verhältnis zwischen Länge und Breite der Perforationslöcher beträgt im ungedehnten Zustand der Folie zumindest 3:2 und kann typischerweise zwischen 3:2 und 4:1 liegen. Durch das Aufschmelzen mittels Laserstrahlung weisen die einzelnen Perforationslöcher üblicherweise eine gleichmäßige ovale Form mit zwei Symmetrieachsen, insbesondere eine in etwa elliptische Form auf. Die Fläche der Perforationslöcher liegt gemäß einer bevorzugten Ausgestaltung der Erfindung zwischen 0,5 mm² und 1 mm², wobei der Lochflächenanteil der Perforation zwischen 0,5 % und 10 % betragen kann.

Die elastische Folie kann grundsätzlich auch nach der Erzeugung der Perforation durch ein Dehnen aktiviert oder bei der Verarbeitung anderweitig gedehnt werden, wobei sich das Verhältnis von 3:2 insbesondere auf die Eigenschaften der elastischen Folie vor einer solchen Aktivierung bzw. an der weitigen Dehnung.

Die Perforationslöcher können in der elastischen Folie gleichmäßig oder in einem Muster vorgesehen sein.

Insbesondere können sich entlang der bevorzugten Dehnrichtung oder senkrecht zu der bevorzugten Dehnrichtung zumindest ein mit Perforationslöchern versehener Streifen und ein unperforierter Streifen abwechseln.

Die Dicke der elastischen Folie liegt typischerweise zwischen 15 µm und 70 µm, wobei die elastische Folie zumindest eine elastische Folienschicht aus einem thermoplastischen Elastomer aufweist. Für eine solche elastische Folienschicht sind beispielsweise elastische Polyolefine oder Styrol-Block-Copolymere wie Styrol-Butadien-Styrol-Block-Copolymer (SBS) und Styrol-Isopren-Styrol-Block-Copolymer (SIS) geeignet.

Als Quelle für die Laserstrahlung kommt insbesondere ein CO2-Laser oder auch ein Nd:YAG-Laser in Betracht. Um gleichzeitig mehrere Perforationslöcher zu erzeugen, kann auf bevorzugte Weise eine Strahlteilung durch entsprechende optische Einrichtungen erfolgen. Um die einzelnen Perforationslöcher mit der erfindungsgemäß langgezogenen Form zu bilden, können die entsprechenden Laserstahlen grundsätzlich auch so aufgefächert werden, dass diese nicht im Wesentlichen punktförmig, sondern linienförmig auf die Folie auftreffen. Bevorzugt ist jedoch vorgesehen, dass zur Erzeugung jedes Perforationsloches ein zugeordneter Laserstrahl im Wesentlichen punkt- oder kreisförmig auf die Folie auftrifft und dann zur Erzeugung der langgezogenen Form über die Folie bewegt wird, was leicht durch piezo-elektrisch oder anderweitig motorisch angetriebene Spiegel möglich ist.

Um die Erzeugung der Perforationslöcher mittels Laser zu erleichtern, kann die elastische Folie auch absorbierende Zusätze enthalten, welche auch als Laseradditive bezeichnet werden. Entsprechende Zusätze können insbesondere bei thermoplastischen Elastomeren auf Olefin-Basis (TPE-O) zweckmäßig sein, um eine ausreichende Absorption der Laserstrahlung sicherzustellen.

Grundsätzlich kann die elastische Folie als Monofolie ausgestaltet sein, die dann ausschließlich aus der beschriebenen elastischen Folienschicht besteht. Alternativ ist auch eine mehrschichtige Ausgestaltung möglich, wobei eine solche Folie insbesondere durch Koextrusion gebildet werden kann.

Bei einer mehrschichtigen Ausgestaltung kann insbesondere auch eine nicht elastische oder weniger elastische Deckschicht vorgesehen sein, wobei jedoch die zumindest eine elastische Folienschicht die gewünschte elastische Rückstellung nach der Dehnung bewirkt. Nicht elastische Deckschichten sind aus dem Stand der Technik bekannt und können beispielsweise aus Polyolefin gebildet sein. Es ergibt sich der Vorteil, dass eine solche elastische Folie zunächst leichter verarbeitet werden kann, wobei in einem aufgerollten Zustand auch ein Verblocken der Folie vermieden wird.

Die Bruchdehnung entlang der bevorzugten Dehnrichtung beträgt vorzugsweise zumindest 600 %, beispielsweise zwischen 700 % und 1.000 %.

Die Bruchdehnung senkrecht zu der bevorzugten Dehnrichtung liegt typischerweise zwischen 110 % und 300 %. Dabei ist zu berücksichtigen, dass die elastische Folie auch senkrecht zu der bevorzugten Dehnrichtung noch in einem gewissen Maße belastbar sein muss, um insbesondere auch eine zuverlässige Verarbeitung zu ermöglichen. Dies gilt insbesondere dann, wenn gemäß einer bevorzugten Ausgestaltung der Erfindung die bevorzugte Dehnrichtung senkrecht zu einer Herstellungs- und Verarbeitungsrichtung ausgerichtet ist.

Bei der Verarbeitung der Folie müssen alleine für den Transport der Folie entlang der Verarbeitungsrichtung Zugkräfte ausgeübt werden, wobei dann eine übermäßige Dehnung sowie eine damit verbundene Verlängerung vermieden und auch ein Zerreißen verhindert werden muss. Eine ausreichende Zugfestigkeit liegt insbesondere dann vor, wenn bei einer typischen Dehnung der Folie entlang der Verarbeitungsrichtung von 5 % bezogen auf eine Ausgangslänge im unbelasteten Zustand die Zugkraft zumindest 1,5 N/Inch (Newton pro 2,54 cm Folienbreite), bevorzugt zumindest 2,5 N/Inch, beispielsweise zwischen 2,5 N/Inch und 3 N/Inch beträgt.

Die Herstellungs- und Verarbeitungsrichtung wird auch als Maschinenrichtung MD bezeichnet, sodass dann die bevorzugte Dehnrichtung der Querrichtung CD entspricht.

Die um die Perforationslöcher vorhandene Wulst weist eine erhöhte Dicke auf, wobei die Dicke beispielsweise um einen Faktor 2 bis 4 größer sein kann als die eigentliche Foliendicke. Daraus ergibt sich auch, dass die angegebene Dicke der elastischen Folie von typischerweise zwischen 15 µm und 70 µm in einem Abstand zu den Perforationen dort zu bestimmen ist, wo die elastische Folie durch die Bildung der Perforationslöcher mittels Laser weitgehend unbeeinflusst bleibt. Die Dicke der Wulst ist um den Umfang der einzelnen Perforationslöcher vorzugsweise gleichbleibend oder in etwa gleichbleibend.

Gegenstand der Erfindung ist auch ein Verfahren zur Herstellung eines elastischen Schichtmaterials, wobei eine elastische Folie entlang einer Produktionsrichtung zugeführt wird und wobei mittels Laserstrahlung eine Vielzahl von Perforationslöchern derart gebildet wird, dass zur Erzeugung jedes Perforationsloches ein zugeordneter Laserstrahl über eine Länge von zumindest 200 µm, vorzugsweise zumindest 300 µm in einer Relativbewegung entlang einer bevorzugten Dehnrichtung der Folie über die elastische Folie bewegt wird und die Perforationslöcher dadurch eine entlang der bevorzugten Dehnrichtung langgezogene Form erhalten.

Gemäß einer bevorzugten Weiterbildung der Erfindung ist vorgesehen, dass die bevorzugte Dehnrichtung senkrecht zu der Produktionsrichtung entlang einer Querrichtung verläuft, wobei die elastische Folie in Querrichtung bei der Erzeugung der Perforation frei von Zugkräften ist. Des Weiteren sind auch entlang der Längsrichtung üblicherweise nur geringe Zugkräfte vorgesehen, bei der die elastische Folie um typischerweise weniger als 20 % gedehnt ist.

Wenn gemäß einer Variante der Erfindung die elastische Folie zumindest eine nicht elastische Deckschicht aufweist, kann auch bei der Produktion eine unerwünschte Dehnung in Längsrichtung durch die übliche Bahnspannung ausgeschlossen werden.

Bei der Herstellung des elastischen Schichtmaterials kann die elastische, mit der Perforation versehene Folie auch mit zumindest einer Deckschicht aus Nonwoven kaschiert werden, wobei besonders bevorzugt an beiden Seiten der elastischen Folie jeweils eine Deckschicht aus Nonwoven vorgesehen ist.

Demnach ist bevorzugt vorgesehen, dass die elastische Folie vor der Verbindung mit zumindest einer Deckschicht mit der beschriebenen Perforation versehen wird. Grundsätzlich kommt aber auch die Erzeugung der Perforation nach der Verbindung von Folie und zumindest einer Deckschicht in Betracht. Eine solche Vorgehensweise kann insbesondere dann zweckmäßig sein, wenn die Folie aufgrund der eingesetzten Materialien und/oder durch laserabsorbierende Zusätze die auftreffende Laserstrahlung stärker absorbiert als eine Deckschicht aus Nonwoven, welche bevorzugt nicht oder nicht wesentlich durch die Lasereinstrahlung modifiziert wird. Wenn lediglich an einer Seite der Folie eine Deckschicht vorgesehen ist, kann eine Bestrahlung mittels Laser zur Erzeugung der Perforation in der Folie auch von der gegenüberliegenden Seite erfolgen.

Grundsätzlich kann die Bildung eines solchen Laminates aus elastischer Folie und mindestens einer Deckschicht aus Nonwoven auf unterschiedliche Art und Weise erfolgen.

Besonders bevorzugt werden die elastische Folie und die zumindest eine Deckschicht aus Nonwoven durch Ultraschall-Schweißen an Verbindungsflächen bzw. Verbindungspunkten miteinander verbunden.

Entsprechende Laminate sind beispielsweise aus der EP 1 355 604 B3 bekannt, wobei gemäß diesem Stand der Technik direkt an den Verbindungsflächen bzw. Verbindungspunkten Poren gebildet werden, um eine Atmungsaktivität zu erzeugen. Entsprechende Poren stellen jedoch im Hinblick auf die Reißfestigkeit des Laminates Schwachstellen dar, sodass das Laminat bei einer Dehnung relativ schnell zerreißt und somit auch nur vergleichsweise geringe Werte für die Bruchdehnung erreicht werden können.

Im Rahmen der Erfindung wird das Ultraschall-Schweißen vor diesem Hintergrund vorzugsweise so durchgeführt, dass an den Verbindungsflächen bzw. Verbindungspunkten keine zusätzlichen Poren erzeugt werden. Insbesondere kann vorgesehen sein, dass trotz des Ultraschall-Schweißens auch an den Verbindungsflächen bzw. Verbindungspunkten die elastische Folie als durchgängige Materialschicht erhalten bleibt.

Im Rahmen einer solchen Erfindung wird also entgegen der EP 1 355 604 B3 die Erzeugung der Perforation von der Bildung der Verbindungsflächen bzw. Verbindungspunkte vorzugsweise funktionell getrennt, sodass insgesamt sehr gute Dehneigenschaften und insbesondere auch ein hoher Wert für die Bruchdehnung bei einer Dehnung entlang der bevorzugten Dehnungsrichtung erzielt werden können.

Wenn im Rahmen einer solchen Ausgestaltung die zumindest eine Deckschicht aus Nonwoven im Wesentlichen oberflächlich mit der elastischen Folie verbunden ist, so ist es im Hinblick auf die mechanischen Eigenschaften auch hinzunehmen, wenn die Perforationslöcher bereichsweise mit den Verbindungsflächen bzw. Verbindungspunkten zusammenfallen oder damit überlappen.

Gemäß einer Weiterbildung der Erfindung ist vorgesehen, dass die elastische Folie nach der Erzeugung der Perforation entlang der bevorzugten Dehnungsrichtung gedehnt und im gedehnten Zustand mit der zumindest einen Deckschicht aus Nonwoven verbunden wird. Es wird damit ein Laminat gebildet, welches nach einem Wegfall der Zugspannung leicht dehnbar ist, selbst wenn das Nonwoven-Material selbst nicht elastisch ist.

Die Erfindung wird im Folgenden anhand einer lediglich ein Ausführungsbeispiel darstellenden Zeichnung erläutert. Es zeigen:
- Fig. 1: einen Ausschnitt aus einer elastischen Folie mit einer von Perforationslöchern gebildeten Perforation,
- Fig. 2: die Detailansicht eines einzelnen Perforationsloches in einer Draufsicht,
- Fig. 3: einen Querschnitt durch ein Perforationsloch,
- Fig. 4A: ein Schichtmaterial mit Streifen der elastischen Folie in einem Querschnitt,
- Fig. 4B: das Schichtmaterial gemäß der Fig. 4A in einer Draufsicht,
- Fig. 5A und Fig. 5B: das Material gemäß der Fig. 4A bzw. der Fig. 4B im gedehnten Zustand,
- Fig. 6: einen Schnitt durch das Schichtmaterial gemäß der Fig. 4A in einer Detailansicht.

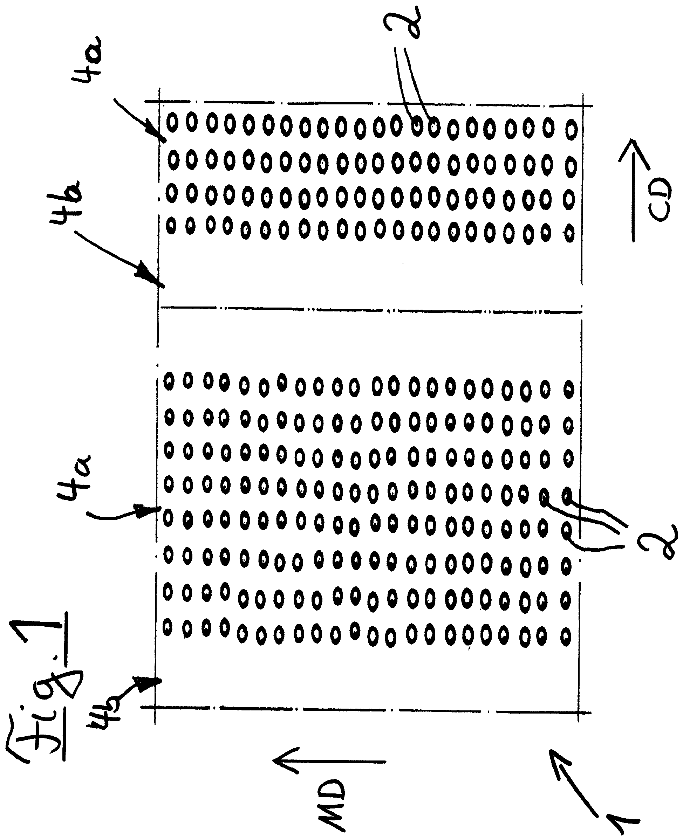

Die Fig. 1 zeigt eine elastische Folie 1 mit einer von Perforationslöchern 2 gebildeten Perforation in einem ungedehnten Zustand. Die elastische Folie 1 weist eine auch als Maschinenrichtung MD bezeichnete Herstellungs- und Verabeitungsrichtung sowie senkrecht dazu eine Querrichtung CD auf.

Wie auch anhand der nachfolgend erläuterten Figuren erkennbar ist, sind die Perforationslöcher 2 mittels Laserstrahlung erzeugt. Gemäß der Fig. 1 ist bereits zu erkennen, dass die Perforationslöcher 2 in dem dargestellten ungedehnten Zustand der elastischen Folie 1 langgezogen entlang der Querrichtung CD sind, wobei die Querrichtung CD auch einer bevorzugten Dehnrichtung der elastischen Folie 1 entspricht. Die gemäß ASTM 882-12 bestimmte Bruchdehnung (elongation at break) ist entlang der Querrichtung CD als bevorzugte Dehnrichtung zumindest doppelt so groß wie die senkrecht dazu entlang der Maschinenrichtung MD bestimmte Bruchdehnung.

Die entlang der Querrichtung CD bestimmte Bruchdehnung beträgt vorzugsweise zumindest 600 %, beispielsweise zwischen 750 % und 1.000 %, insbesondere etwa 800 %.

Die entlang der Maschinenrichtung MD bestimmte Bruchdehnung liegt typischerweise zwischen 110 % und 300 %.

Dieses stark anisotrope Verhalten ist auch auf die langgezogene Form der Perforationslöcher 2 zurückzuführen bzw. wird dadurch begünstigt. Bereits aus der Fig. 1 ist zu erkennen, dass die entlang der Querrichtung CD langgezogenen Perforationslöcher 2 im Hinblick auf diese bevorzugte Dehnrichtung eine vergleichsweise geringe Schwächung darstellen.

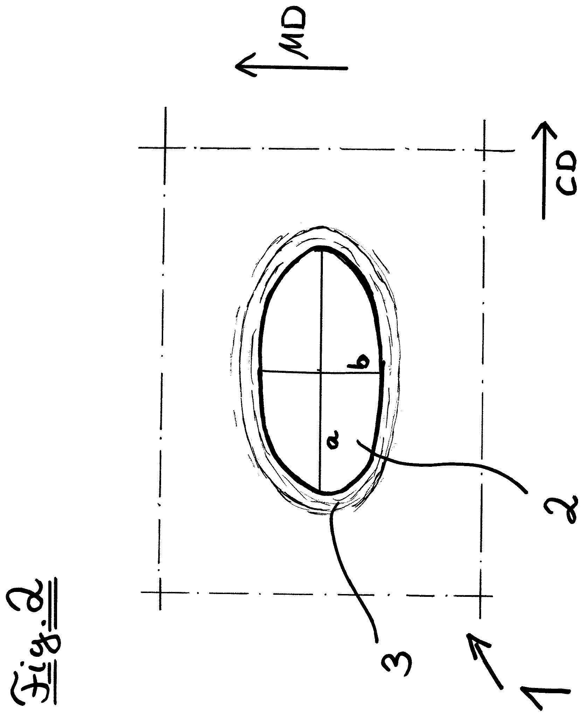

Um die entlang der Querrichtung CD langgezogene Form der einzelnen Perforationslöcher 2 zu erreichen, wird zur Erzeugung jedes Perforationsloches 2 ein zugeordneter Laserstrahl über eine bestimmte Länge von vorzugsweise zumindest 200 µm in einer Relativbewegung entlang der Querrichtung CD als bevorzugte Dehnrichtung der Folie 1 über die elastische Folie 1 bewegt, sodass dann gemäß der Fig. 2 die einzelnen Perforationslöcher 2 durch das Aufschmelzen des Polymermaterials eine ovale, insbesondere etwa elliptische Form mit einer langen Achse a entlang der Querrichtung CD und eine kurze Achse b entlang der Maschinenrichtung MD aufweisen.

Das Verhältnis der entlang der langen Achse a bestimmten Länge zu der entlang der kurzen Achse b bestimmten Breite der Perforationslöcher 2 beträgt zumindest 3:2 und besonders bevorzugt etwa 2:1. Insbesondere kann das Verhältnis zwischen 3:2 und 4:1 betragen.

Die Fläche der einzelnen Perforationslöcher 2 liegt typischerweise zwischen 0,05 mm² und 1 mm², vorzugsweise zwischen 0,1 mm² und 0,5 mm² und besonders bevorzugt zwischen 0,15 mm² und 0,3 mm².

Der gesamte Lochflächenanteil der Perforation in Bezug auf die Gesamtfläche der elastischen Folie 1 kann insbesondere zwischen 0,5 % und 10 % liegen.

Durch die von den Perforationslöchern 2 gebildete Perforation weist die elastische Folie 1 eine ausreichende Atmungsaktivität auf, wobei erfindungsgemäß die elastischen Dehnungseigenschaften entlang der Querrichtung CD nur geringfügig beeinflusst werden. Insbesondere kann eine hohe Bruchdehnung erreicht werden, die mit den Eigenschaften einer unperforierten Folie vergleichbar ist, während entlang der Maschinenrichtung MD eine deutliche Reduzierung der Bruchdehnung in Kauf genommen werden kann.

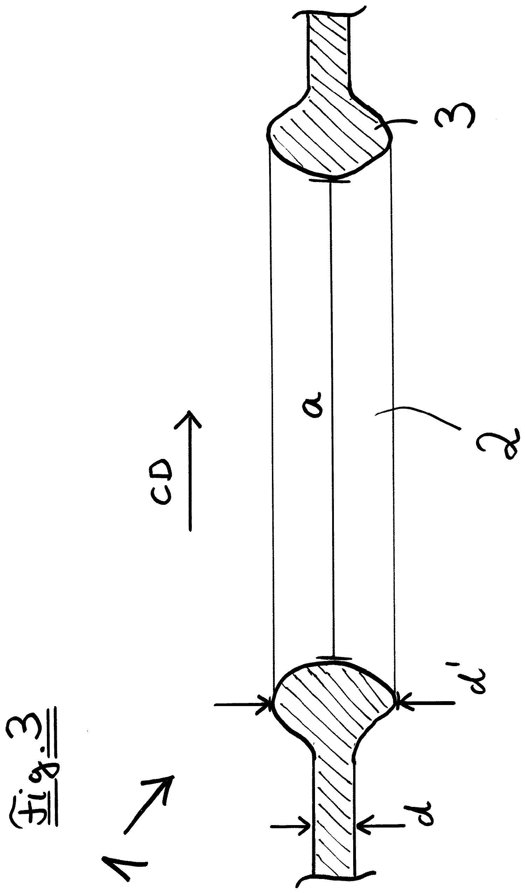

Gemäß der Fig. 3 trägt dazu auch wesentlich bei, dass bei der Erzeugung der Perforationslöcher 2 mittels Laser das aufgeschmolzene Polymermaterial um das eigentliche Perforationsloch 2 herum eine Wulst 3 bildet. Ausgehend von einer Dicke d der elastischen Folie 1 von typischerweise zwischen 15 µm und 70 µm weist die Wulst 3 eine um zumindest einen Faktor 2 erhöhte Dicke d' auf.

In dem dargestellten Ausführungsbeispiel kann die Dicke d der elastischen Folie 1 außerhalb der Perforation 2 und der Wulst 3 beispielsweise 50 µm betragen, während die Dicke d' an der Wulst 3 dann beispielsweise 150 µm beträgt.

Die Wulst 3 trägt somit zu einer wesentlichen Verstärkung und Stabilisierung bei, wobei sich auch ein besonders gleichmäßiger und glatter Rand ergibt, der einem Einreißen widerstehen kann.

Die von den Perforationslöchern 2 gebildete Perforation kann gleichmäßig über die gesamte elastische Folie 1 angeordnet sein. Die Fig. 1 zeigt eine Ausgestaltung, bei der sich entlang der Querrichtung CD als bevorzugter Dehnrichtung mit den Perforationslöchern 2 versehene Streifen 4a und unperforierte Streifen 4b, die jeweils entlang der Maschinenrichtung MD verlaufen, abwechseln. Wie nachfolgend noch weiter beschrieben, können einzelne entlang der Maschinenrichtung MD verlaufende Abschnitte 5 dadurch gebildet werden, dass die elastische Folie 1 mittig an den unperforierten Streifen 4b geschnitten wird, wobei dann diese einzelnen Abschnitte 5 zur Bildung eines elastischen Schichtmaterials eingesetzt werden können.

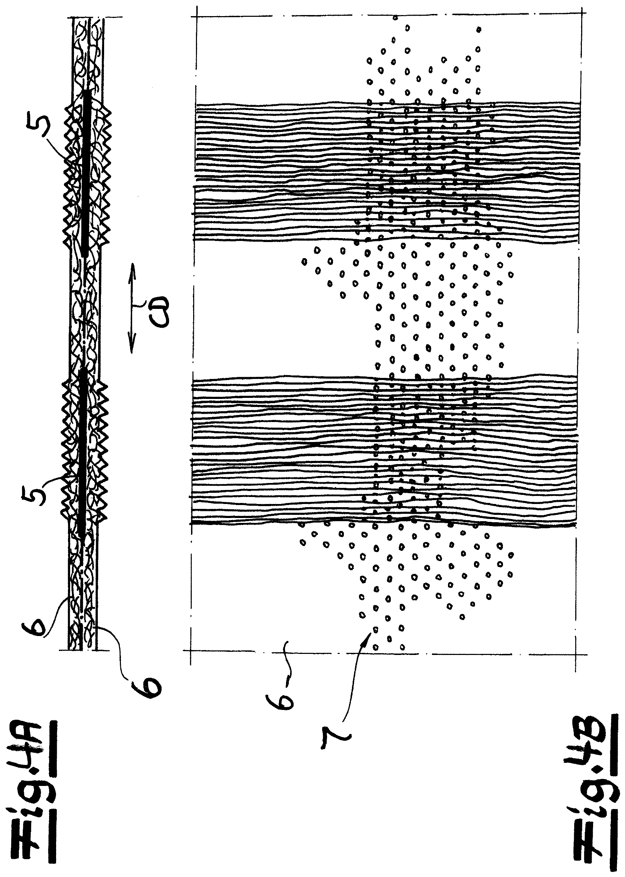

Die Fig. 4A zeigt in diesem Zusammenhang ein Laminat mit Deckschichten 6 aus Nonwoven und mit Abschnitten 5 der Folie 1 dazwischen. Wie zuvor beschrieben, weisen die Abschnitte 5 die von den Perforationslöchern 2 gebildete Perforation auf, wobei aber jeweils die Ränder der Abschnitte 5 unperforiert sind und so leicht verarbeitet werden können.

Die elastische Folie 1 kann ohne Einschränkung als Monofolie oder mehrschichtig als koextrudierte Folie gebildet sein, wobei die elastische Folie 1 zumindest eine elastische Folienschicht aufweist, welche bei einer Dehnung die gewünschte elastische Rückstellung bewirkt.

Gemäß dem in Fig. 4A dargestellten Ausführungsbeispiel ist zu erkennen, dass an den Abschnitten 5 der elastischen Folie 1 die Deckschichten 6 aus Nonwoven entlang der Querrichtung CD wellenförmig zusammengeschoben sind. Dies ist darauf zurückzuführen, dass die Abschnitte 5 der elastischen Folie 1 bei ihrer Verbindung mit den Deckschichten 6 entlang der Querrichtung CD gedehnt sind (stretch-bonding), wobei durch die elastische Rückstellung der unter Zugspannung verbundenen Abschnitte 5 der elastischen Folie 1 nach einem Wegfall der Zugspannung das bei der Verbindung noch flach liegende Nonwoven der Deckschichten 6 entlang der Querrichtung CD komprimiert wird.

Aus dem dargestellten Laminat können einzelne Verschlussbänder derart herausgeschnitten werden, dass an den einzelnen Verschlussbändern im Bereich der elastischen Folie 1 ein elastischer Mittelabschnitt gebildet wird, während die daran anschließenden Enden des Zuschnitts lediglich aus den beiden miteinander verbundenen Deckschichten 6 gebildet und unelastisch sind.

Die Deckschichten 6 und die elastische Folie 1 sind in dem dargestellten Ausführungsbeispiel durch Ultraschall-Schweißen lediglich partiell durch ein Muster aus Verbindungspunkten 7 miteinander verbunden. Im Vergleich zu einer Kaschierung mittels Klebstoff ergibt sich der Vorteil, dass durch das Ultraschall-Schweißen kein weiteres Material in das Laminat eingebracht werden muss, was auch hinsichtlich eines Recyclings oder auch einer Geruchsentwicklung des Laminates von Vorteil sein kann.

Da die Verbindung der Deckschichten 6 mit der elastischen Folie 1 im gedehnten Zustand der in Form von Abschnitten 5 zugeführten elastischen Folie 1 erfolgt, liegen die Verbindungspunkte 7 im Bereich der elastischen Folie 1 auch enger beieinander als in den übrigen Bereichen, an denen die beiden Deckschichten 6 direkt durch die Verbindungspunkte 7 miteinander verbunden sind.

Durch die Verbindung der elastischen Folie 1 mit den Deckschichten 6 im gedehnten Zustand ist das so gebildete Laminat bis zu der Ausdehnung der Folie 1 während des Verbindungsprozesses im Nachfolgenden leicht dehnbar, wobei dann die mechanischen Eigenschaften im Wesentlichen nur von der Elastizität der elastischen Folie 1 bestimmt werden.

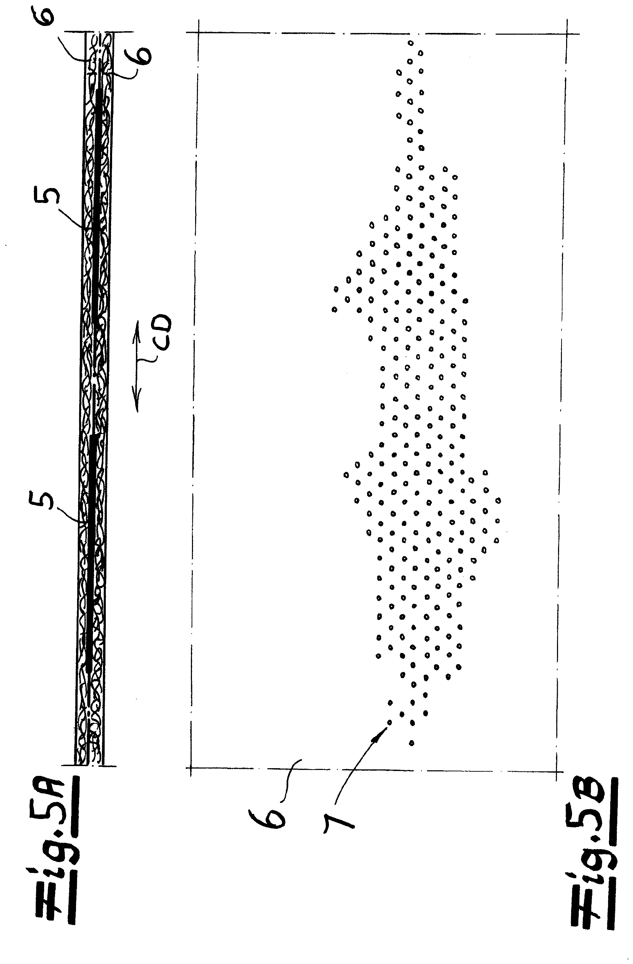

Die Figuren 5A und 5B zeigen entsprechend der Darstellung der Figuren 4A und 4B das Laminat in einem gedehnten Zustand, wobei dann die zuvor im ungedehnten Zustand im Bereich der elastischen Folie 1 wellenförmig zusammengeschobenen Deckschichten 6 wieder flach verlaufen.

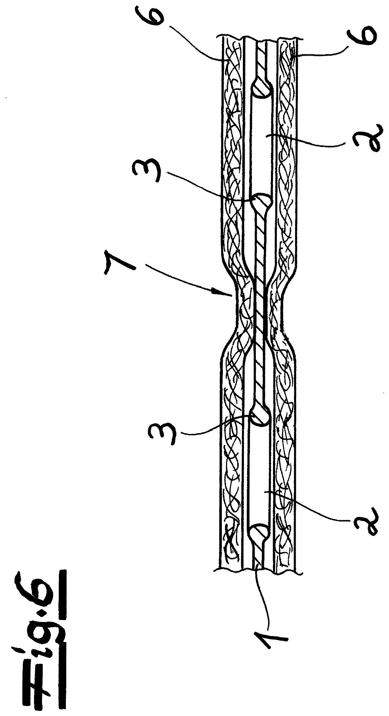

Um die Atmungsaktivität des Laminates auch im Bereich der elastischen Folie zu erreichen, ist die elastische Folie 1 wie zuvor beschrieben mit den Perforationslöchern 2 versehen. Zur besseren Erkennbarkeit sind in der Fig. 6 die Perforationslöcher 2 in einem Abstand zu den Verbindungspunkten 7 dargestellt, wobei sich die Verbindungspunkte 7 auch mit den Perforationslöchern 2 überschneiden können.

Im Rahmen der dargestellten Ausführungsform ist es jedoch wesentlich, dass die elastische Folie 1 an den Verbindungspunkten 7 als durchgängige Schicht erhalten bleibt und dort keine zusätzlichen Poren oder Öffnungen erzeugt werden. Entsprechend resultiert auch dort keine wesentliche Materialschwächung, wobei lediglich die mittels Laser gebildeten Perforationslöcher 2 die gewünschte Atmungsaktivität bereitstellen.

Wie bereits zuvor beschrieben, kann durch die Form der Perforationslöcher 2 eine sehr gute Dehnbarkeit entlang der Querrichtung CD als bevorzugter Dehnrichtung erreicht werden, wobei gemäß der Fig. 6 auch die Verbindungspunkte 7 nicht zu einer unerwünschten Schwächung des Materials führen.

## Patentansprüche

1. Elastische Folie (1) mit einer von Perforationslöchern (2) gebildeten Perforation, **dadurch gekennzeichnet, dass** die Perforationslöcher (2) in einem ungedehnten Zustand entlang einer bevorzugten Dehnrichtung langgezogen sind, wobei das Verhältnis der entlang der bevorzugten Dehnrichtung bestimmten Länge zu einer senkrecht dazu bestimmten Breite der Perforationslöcher (2) zumindest 3:2 beträgt und dass die Bruchdehnung entlang der bevorzugten Dehnrichtung zumindest doppelt so groß ist, wie die senkrecht dazu entlang der Breite der Perforationslöcher (2) bestimmte Bruchdehnung.

2. Elastische Folie (1) nach Anspruch 1, **dadurch gekennzeichnet, dass** die Perforationslöcher (2) jeweils eine Fläche zwischen 0,05 mm² und 1 mm² aufweisen.

3. Elastische Folie (1) nach Anspruch 1 oder 2, **dadurch gekennzeichnet, dass** die Dicke (d) zwischen 15 µm und 70 µm beträgt.

4. Elastische Folie (1) nach einem der Ansprüche 1 bis 3, **dadurch gekennzeichnet, dass** um die Perforationslöcher (2) jeweils eine Wulst (3) mit einer erhöhten Dicke (d') verläuft.

5. Elastische Folie (1) nach einem der Ansprüche 1 bis 4, **dadurch gekennzeichnet, dass** die Bruchdehnung entlang der bevorzugten Dehnrichtung zumindest 600 % beträgt.

6. Elastische Folie (1) nach einem der Ansprüche 1 bis 5, **dadurch gekennzeichnet, dass** die Bruchdehnung senkrecht zu der bevorzugten Dehnrichtung zwischen 110 % und 300 % liegt.

7. Elastische Folie (1) nach einem der Ansprüche 1 bis 6, **dadurch gekennzeichnet, dass** sich entlang der bevorzugten Dehnrichtung oder senkrecht zu der bevorzugten Dehnrichtung zumindest ein mit Perforationslöchern (2) versehener Streifen (4a) und ein unperforierter Streifen (4b) abwechseln.

8. Elastische Folie (1) nach einem der Ansprüche 1 bis 7, **dadurch gekennzeichnet, dass** die bevorzugte Dehnrichtung senkrecht zu einer Herstellungs- und Verarbeitungsrichtung ausgerichtet ist.

9. Elastische Folie (1) nach einem der Ansprüche 1 bis 8, **dadurch gekennzeichnet, dass** zumindest eine elastische Folienschicht aus Styrol-Block-Copolymer gebildet ist.

10. Elastische Folie (1) nach einem der Ansprüche 1 bis 9, **dadurch gekennzeichnet, dass** der Lochflächenanteil der Perforation zwischen 0,5 % und 10 % beträgt.

11. Verfahren zur Herstellung eines elastischen Schichtmaterials
wobei eine elastische Folie (1) entlang einer Produktionsrichtung zugeführt wird und
wobei mittels Laserstrahlung eine Vielzahl von Perforationslöchern (2) derart gebildet wird, dass zur Erzeugung jedes Perforationsloches (2) ein zugeordneter Laserstrahl über eine Länge von zumindest 200 µm in einer Relativbewegung entlang einer bevorzugten Dehnrichtung der elastischen Folie (1) über die elastische Folie (1) bewegt wird und die Perforationslöcher (2) dadurch eine entlang der bevorzugten Dehnrichtung langgezogene Form halten, wobei es sich bei der elastischen Folie (1) um eine elastische Folie (1) gemäss Anspruch 1 handelt.

12. Verfahren nach Anspruch 11, wobei die bevorzugte Dehnrichtung senkrecht zu der Produktionsrichtung entlang einer Querrichtung (CD) verläuft und die elastische Folie (1) in Querrichtung (CD) bei der Erzeugung der Perforation frei von Zugkräften ist.

13. Verfahren nach Anspruch 11 oder 12, wobei die elastische, mit der Perforation versehene Folie (1) mit zumindest einer Deckschicht (6) aus Nonwoven kaschiert wird.

14. Verfahren nach Anspruch 13, wobei die elastische Folie (1) und die zumindest eine Deckschicht (6) aus Nonwoven durch Ultraschall-Schweißen an Verbindungsflächen bzw. Verbindungspunkten (7) miteinander verbunden werden.

15. Verfahren nach Anspruch 13 oder 14, wobei die elastische Folie (1) nach der Erzeugung der Perforation entlang der bevorzugten Dehnrichtung gedehnt und im gedehnten Zustand mit der zumindest einen Deckschicht (6) aus Nonwoven verbunden wird.

## Claims

1. Elastic film (1) having a perforation formed by perforation holes (2), **characterized in that** the perforation holes (2) are elongated along a preferred stretching direction in an unstretched state, wherein the ratio of the length determined along the preferred stretching direction to a width of the perforation holes (2) determined perpendicular thereto is at least 3:2 and that the elongation at break along the preferred stretching direction is at least twice as great as the elongation at break determined perpendicular thereto along the width of the perforation holes (2).

2. Elastic film (1) according to Claim 1, **characterized in that** the perforation holes (2) each have an area of between 0.05 mm² and 1 mm².

3. Elastic film (1) according to Claim 1 or 2, **characterized in that** the thickness (d) is between 15 µm and 70 µm.

4. Elastic film (1) according to one of Claims 1 to 3, **characterized in that** a protuberance (3) having an increased thickness (d') runs around the perforation holes (2).

5. Elastic film (1) according to one of Claims 1 to 4, **characterized in that** the elongation at break along the preferred stretching direction is at least 600 %.

6. Elastic film (1) according to one of Claims 1 to 5, **characterized in that** the elongation at break perpendicular to the preferred stretching direction is between 110 % and 300 %.

7. Elastic film (1) according to one of Claims 1 to 6, **characterized in that** at least one strip (4a) provided with perforation holes (2) and an unperforated strip (4b) alternate along the preferred stretching direction or perpendicular to the preferred stretching direction.

8. Elastic film (1) according to one of Claims 1 to 7, **characterized in that** the preferred stretching direction is aligned perpendicular to a production and processing direction.

9. Elastic film (1) according to one of Claims 1 to 8, **characterized in that** at least one elastic film layer is formed from styrene block copolymer.

10. Elastic film (1) according to one of Claims 1 to 9, **characterized in that** the hole area percentage of the perforation is between 0.5 % and 10 %.

11. Method for producing an elastic layer material,
wherein an elastic film (1) is supplied along a production direction and
wherein a plurality of perforation holes (2) is formed by means of laser radiation in such a manner that to produce each perforation hole (2), an associated laser beam is moved over the elastic film (1) over a length of at least 200 µm in a relative movement along a preferred stretching direction of the elastic film (1) and the perforation holes (2) thereby obtain a shape that is elongated along the preferred stretching direction, wherein the elastic film (1) is an elastic film (1) according to Claim 1.

12. Method according to Claim 11, wherein the preferred stretching direction runs perpendicular to the production direction along a transverse direction (CD) and the elastic film (1) is free from tensile forces in the transverse direction (CD) during production of the perforation.

13. Method according to Claim 11 or 12, wherein the elastic film (1) provided with the perforation is laminated with at least one cover layer (6) of nonwoven.

14. Method according to Claim 13, wherein the elastic film (1) and the at least one cover layer (6) of nonwoven are connected to one another at connection surfaces or connection points (7) by ultrasonic welding.

15. Method according to Claim 13 or 14, wherein the elastic film (1) is stretched along the preferred stretching direction after producing the perforation and in the stretched state is connected to the at least one cover layer (6) of nonwoven.

## Revendications

1. Film élastique (1) comprenant une perforation formée par des trous de perforation (2), **caractérisé en ce que** les trous de perforation (2) sont étirés en longueur dans un état non allongé le long d'un sens d'allongement préféré, dans lequel le rapport de la longueur déterminée le long du sens d'allongement préféré à une largeur des trous de perforation (2) déterminée verticalement en rapport est d'au moins 3:2 et que l'allongement à la rupture le long du sens d'allongement préféré est au moins deux fois l'allongement à la rupture déterminé verticalement en rapport le long de la largeur des trous de perforation (2).

2. Film élastique (1) selon la revendication 1, **caractérisé en ce que** les trous de perforation (2) présentent respectivement une surface entre 0,05 mm² et 1 mm².

3. Film élastique (1) selon la revendication 1 ou 2, **caractérisé en ce que** l'épaisseur (d) fait entre 15 µm et 70 µm.

4. Film élastique (1) selon l'une des revendications 1 à 3, **caractérisé en ce qu'**un bourrelet (3) avec une épaisseur accrue (d') entoure les trous de perforation (2) .

5. Film élastique (1) selon l'une des revendications 1 à 4, **caractérisé en ce que** l'allongement à la rupture le long du sens d'allongement préféré est au moins de 600 %.

6. Film élastique (1) selon l'une des revendications 1 à 5, **caractérisé en ce que** l'allongement à la rupture verticalement au sens d'allongement préféré est entre 110 % et 300 %.

7. Film élastique (1) selon l'une des revendications 1 à 6, **caractérisé en ce que** le long du sens d'allongement préféré ou verticalement au sens d'allongement préféré, au moins une bande (4a) dotée de trous de perforation (2) et une bande (4b) non perforée s'alternent.

8. Film élastique (1) selon l'une des revendications 1 à 7, **caractérisé en ce que** le sens d'allongement préféré est aligné verticalement à un sens de fabrication et de traitement.

9. Film élastique (1) selon l'une des revendications 1 à 8, **caractérisé en ce qu'**au moins une couche de film élastique en copolymère bloc styrène est formée.

10. Film élastique (1) selon l'une des revendications 1 à 9, **caractérisé en ce que** la part de surface à trous de la perforation fait entre 0,5 % et 10 %.

11. Procédé destiné à la fabrication d'un matériau en couche élastique
dans lequel un film élastique (1) est dirigé le long d'un sens de production et
dans lequel par rayonnement laser, une pluralité de trous de perforation (2) sont ainsi formés que pour produire chaque trou de perforation (2), un faisceau laser correspondant est déplacé sur le film élastique (1) sur une longueur d'au moins 200 µm dans un mouvement relatif le long d'un sens d'allongement préféré du film élastique (1), et les trous de perforation (2) maintiennent ainsi une forme étirée en longueur le long du sens d'allongement préféré, dans lequel le film élastique (1) est un film élastique (1) selon la revendication 1.

12. Procédé selon la revendication 11, dans lequel le sens d'allongement préféré est vertical au sens de production le long d'un sens transversal (CD) et le film élastique (1) dans le sens transversal (CD) est exempt de forces de traction lors de la production de la perforation.

13. Procédé selon la revendication 11 ou 12, dans lequel le film élastique (1) doté de la perforation est masqué par au moins une couche de dessus (6) en non-tissé.

14. Procédé selon la revendication 13, dans lequel le film élastique (1) et l'au moins une couche de dessus (6) en non-tissé sont reliés entre eux par un soudage aux ultra-sons sur des zones de liaison ou des points de liaison (7).

15. Procédé selon la revendication 13 ou 14, dans lequel le film élastique (1), après la fabrication de la perforation est allongé le long du sens d'allongement préféré et relié à l'au moins une couche de dessus (6) en non-tissé dans l'état allongé.
